# EUROPÄISCHE PATENTANMELDUNG

(11) **EP 3 738 560 A1**
(43) Veröffentlichungstag der Anmeldung: **18.11.2020**
(21) Anmeldenummer: 20172747.6
(22) Anmeldetag: 04.05.2020
(51) Int. Cl.: A61F 13/00

(54) **VERBAND**

(30) Priorität: 16.05.2019 CH 6412019
(71) Anmelder: Laedi Ortho AG, 6010 Kriens (CH)
(72) Erfinder: LÄDERACH, Heinz, 4900 Langenthal (CH)
(74) Vertreter: Keller Schneider Patent- und Markenanwälte AG (Bern)

(57) **Zusammenfassung**

Ein Verband (10), insbesondere zur Verwendung in einem Erste-Hilfe Einsatz oder als Stützverband, umfassend ein Bandteil (11), an welchem zumindest bereichsweise eine medizinische, insbesondere eine antiseptische Substanz fixiert ist. Weiter betrifft die Erfindung ein Verfahren zur Herstellung eines solchen Verbandes (10).

## Beschreibung

### Technisches Gebiet

Die Erfindung betrifft einen Verband, insbesondere zur Verwendung in einem Erste-Hilfe Einsatz oder als Stützverband, umfassend einen Bandteil.

### Stand der Technik

Verbände werden in der Medizin und Therapie seit langem eingesetzt. In medizinischen Anwendungen werden Verbände zur Befestigung von Wundauflagen am Körper, als Schutz vor Umwelteinflüssen, zur Ruhigstellung, zur Fixierung von Salben oder wirkstoffgetränkten Polstern, zur Blutstillung und Sekretaufnahme etc. eingesetzt. In therapeutischen Anwendungen dient der Verband zum Beispiel zum Stützen von Gelenken, zur Kompressionstherapie etc.

Die bekannten Verbänden haben den Nachteil, dass deren Einsatz aufwändig und kompliziert ist.

Die bekannten Verbände haben insbesondere weiter den Nachteil, dass trotz grosser Sorgfalt in der Anwendung Verunreinigungen und damit Wundinfektionen nicht ausgeschlossen werden können.

### Darstellung der Erfindung

Aufgabe der Erfindung ist es, ein dem eingangs genannten technischen Gebiet zugehörender Verband zu schaffen, womit eine medizinische respektive eine therapeutische Behandlung effizient durchführbar ist und insbesondere ein Risiko der Wundinfektion vermindert werden kann.

Die Lösung der Aufgabe ist durch die Merkmale des Anspruchs 1 definiert. Gemäss der Erfindung ist am Bandteil zumindest bereichsweise eine medizinische, insbesondere eine antiseptische Substanz fixiert.

Durch die Fixierung der medizinischen Substanz am Bandteil wird ein Verband geschaffen, womit eine Behandlung eines Patienten besonders effizient erfolgen kann. Sofern der Patient ausschliesslich mit der medizinischen Substanz behandelt werden soll, kann die Behandlung durch einfaches Anlegen des Verbandes bereits erreicht werden. Insbesondere kann damit auf das Auftragen der medizinischen Substanz auf der Haut oder der Wunde des Patienten verzichtet werden und somit eine effizientere Durchführung der Behandlung, insbesondere der medizinischen respektive der therapeutischen Behandlung, erreicht werden. Damit wird in einem einzigen Arbeitsschritt, namentlich mit dem Anbringen des Verbands, sowohl der betroffene Körperteil mit dem Verband versehen als auch der Körperteil mit der Substanz behandelt.

Unter dem Begriff Verband wird nachfolgend eine streifenförmige Stoffbahn mit gegebenenfalls weiteren Bestandteilen, wie ein oder mehrere Verschlussteile etc. verstanden, welche insbesondere in der Therapie und Medizin zum Einbinden von Körperteilen eingesetzt werden können. Der Verband ist insbesondere bevorzugt zur Verwendung im Erste-Hilfe Einsatz, bei Unfällen im Verkehr, Arbeit etc. ausgelegt. Der Verband kann aber auch bei Tieren eingesetzt werden. Der Verband kann aber auch als Stützverband bei Sportverletzungen, wie z.B. bei Zerrungen, als Blutsperre etc. eingesetzt werden.

Unter dem Bandteil wird nachfolgend die streifenförmige Stoffbahn verstanden, welche den Hauptbestandteil des Verbandes ausmacht. Die Stoffbahn kann mit unterschiedlichen Herstellungsverfahren hergestellt sein (siehe weiter unten).

Bei der medizinischen Substanz handelt es sich vorzugsweise um einen Wirkstoff, welcher perkutan aufgenommen werden kann. Alternativ können aber auch Wirkstoffe vorgesehen sein, welche bei offenen Wunden direkt aufgenommen werden können. Die möglichen Wirkstoffe sind dem Fachmann, insbesondere dem Arzt hinreichend bekannt. Die Wirkstoffen müssen nicht zwingend verschreibungspflichtig sein, sondern können auch zum Beispiel frei erhältliche Pflanzenextrakte und dergleichen umfassen. Besonders bevorzugt handelt es sich bei der medizinischen Substanz um eine antiseptische Substanz. Die antisetpische Wirkung stellt ein Grundbedürfnis in allen Anwendungsbereichen dar, da damit das Infektionsrisiko vermindert werden kann. Der Verband kann zusätzlich zu der antiseptischen Substanz mit weiteren Substanzen versetzt sein.

Die medizinische Substanz, insbesondere die antiseptische Substanz ist am Bandteil fixiert. Mit "am Bandteil" ist gleichermassen eine oberflächliche Fixierung und/oder eine Fixierung innerhalb des Bandteils zu verstehen. Damit wird erreicht, dass die Substanz auch bei einem Waschvorgang nicht, respektive nicht vollständig entfernt werden kann. In einer bevorzugten Ausführungsform ist die Substanz derart fixiert, dass diese auch nach mehr als 10, vorzugsweise mehr als 25 Waschvorgängen noch in wirksamer Menge auf dem Bandteil fixiert ist. Damit kann der Verband mehrfach verwendet werden. Die Fixierung kann mit unterschiedlichen Techniken erreicht werden. Die Fixierung kann chemisch oder physikalisch erfolgen. Einerseits kann ein Wirkstoff über kovalente Bindungen an eine Faser des Bandteils gebunden werden. Weiter kann ein Wirkstoff über starke zwischenmolekulare Kräfte am Bandteil gehalten werden. Weiter kann der Wirkstoff in Poren des Bandteils aufgenommen sein. Der Wirkstoff kann auch in einem Trägermaterial, zum Beispiel in einem Faden oder dergleichen, eingebracht sein. Dem Fachmann sind weitere Varianten bekannt.

Die Substanz, insbesondere die antiseptische Substanz kann zum Beispiel Silberfäden umfassen, welche in den Bandteil eingewoben oder anderweitig am Bandteil fixiert werden. Die Substanz kann weiter Zink und/oder weitere dem Fachmann bekannte Antiseptika umfassen. Der Bandteil kann mittels des dem Fachmann bekannten Foulard-Verfahrens mit den Substanzen imprägniert werden. In Varianten können auch andere Verfahren eingesetzt werden, um den Bandteil mit den Substanzen zu imprägnieren.

Vorzugsweise umfasst der Verband einen streifenförmigen Bandteil und einen Verschlussteil. Mit dem Verschlussteil kann die Befestigung des Verbands am Körper vereinfacht werden. Weiter kann damit der Verband z.B. in aufgerolltem Zustand fixiert werden. Der Verschlussteil kann Haken, Druckknöpfe, Bänder etc. umfassen.

In Varianten kann auf den Verschlussteil verzichtet werden.

Vorzugsweise weist der Verband eine Länge zwischen 500 mm und 2000 mm, insbesondere zwischen 800 mm und 1400 mm, besonders bevorzugt zwischen 900 und 1200 mm auf. Damit kann der Verband für die meisten Anwendungen, insbesondere in der Erste-Hilfe, eingesetzt werden.

In Varianten kann der Verband auch kürzer als 500 mm oder länger als 2000 mm sein.

Bevorzugt liegt eine Breite des Bandteils zwischen 50 mm und 100 mm, insbesondere zwischen 70 mm und 80 mm. Auch hierbei wird mit der Wahl der Breite ein besonders vielfältiger Einsatz ermöglicht.

In Varianten kann die Breite auch kleiner als 50 mm oder grösser als 100 mm sein.

Vorzugsweise ist der Bandteil in einer Längsrichtung dehnbar, insbesondere zwischen 20 % und 100 %, vorzugsweise zwischen 30 % und 70 %, insbesondere bevorzugt zwischen 45 % und 55 % bezogen auf eine Gesamtlänge des Verbandes. Damit wird gewährleistet, dass der Verband in der Verwendung am Körper anliegt, insbesondere auch dann, wenn sich der Patient bewegt. Weiter können mit einem Bandteil in besagtem Dehnungsbereich besonders optimale Kompressionen erreicht werden. Weiter kann das Band auch als Terraband zum Einsatz im Sport eingesetzt werden, insbesondere da es von Kopf bis Fuss befestigt werden kann.

In Varianten kann der Bandteil in Längsrichtung auch weniger als zu 20 % oder mehr als 100 % dehnbar sein.

Vorzugsweise ist der Bandteil Quersteif. Damit wird verhindert, dass sich der Verband bei der Längsdehnung in Querrichtung zusammenzieht. Durch die quersteife Ausbildung des Bandteils wird ein besonders einfach anzubringender Verband erreicht.

In Varianten kann der Bandteil in Querrichtung dehnbar sein. Vorzugsweise ist der Bandteil in Querrichtung weniger dehnbar als in Längsrichtung.

Vorzugsweise umfasst der Bandteil ein Polyamid mit einem Anteil zwischen 80 % und 99 %, insbesondere einem Anteil zwischen 85 % und 95 % und ein Elastomer mit einem Anteil von 1 % bis 20 %, insbesondere zwischen 5 % und 15 %.

In Varianten kann der Bandteil auch aus anderen Polymeren ausgebildet sein. Weiter kann der Bandteil auch organische Materialien, wie Leine, Wolle etc. umfassen.

Bevorzugt umfasst das Elastomer einen Silikonfaden. Silikonfäden haben den Vorteil, dass sie bei 95 °C ausgekocht werden können und zudem vollständig biokompatibel sind. In Varianten kann auf den Silikonfaden verzichtet werden.

Vorzugsweise ist der Bandteil einlagig ausgebildet. Damit wird ein besonders einfacher und kostengünstiger Aufbau des Bandteils erreicht. In Varianten kann der Bandteil auch mehrlagig ausgebildet sein.

Vorzugsweise umfasst der Verschlussteil eine Lasche, welche insbesondere als Daumenlasche und vorzugsweise längselastisch ausgebildet ist. Mit der Lasche kann der Verband zu Beginn des Anbringens an einem Körperteil, zum Beispiel an einem Daumen vorbefestigt werden. Damit wird ein besonders einfaches Anbringen des Verbands erreicht.

In Varianten kann auf die Lasche auch verzichtet werden.

Bevorzugt ist die Lasche an einem ersten Längsende des Bandteils angeordnet und weist insbesondere eine Länge zwischen 100 mm und 300 mm, insbesondere zwischen 150 und 190 mm und eine Breite zwischen 5 mm und 30 mm, insbesondere zwischen 10 mm und 14 mm auf. Die Anordnung am ersten Längsende des Bandteils hat den Vorteil, dass der Verband nach der Vorbefestigung über die Lasche einfach angebracht werden kann. Die Lasche ist durch die obige Wahl der Länge und der Breite derart dimensioniert, dass die Lasche sowohl als Daumenlasche, aber auch für weitere Körperteile, insbesondere für Ohren, Hände, Füsse etc. geeignet ist. Die Dimensionierung des Verbandes als Ganzes wurde derart entwickelt, dass der Verband für jede Körpergrösse/jedes Geschlecht eingesetzt werden kann. Damit wird ein multifunktioneller Einsatz des Verbands erreicht.

In Varianten kann die Lasche auch anderweitig dimensioniert sein.

Vorzugsweise umfasst der Verschlussteil weiter eine Klettlasche, welche mit einer geeigneten Oberflächenstruktur des Verbands zusammen wirken kann, um einen Verschluss des Verbands zu ermöglichen. Die Oberflächenstruktur ist dem Fachmann hinreichend bekannt. Kommerziell ist eine geeignete Oberflächenstruktur unter dem Begriff VELCRO © bekannt.

In Varianten kann auf den Klettverschluss auch verzichtet werden. Dem Fachmann sind weitere Verschlussteile bekannt, welche zum Befestigen des Verbandes eingesetzt werden können.

Vorzugsweise ist eine erste Hauptfläche des Bandteils derart ausgebildet, dass sie mit der Klettlasche zusammen wirken kann, wobei die erste Hauptfläche insbesondere faserig ausgebildet ist, und eine der ersten gegenüberliegende, zweite Hauptfläche derart ausgebildet ist, dass sie nicht mit der Klettlasche zusammen wirken kann, wobei die zweite Hauptfläche insbesondere glatt ausgebildet ist. Vorzugsweise ist die gesamte erste Hauptfläche faserig ausgebildet, so dass die Klettlasche an beliebiger Stelle mit der ersten Hauptfläche verbunden werden kann.

In Varianten kann auch nur ein Endbereich des Bandteils faserig ausgebildet sein. Weiter kann der Bandteil auf der ersten Hauptfläche auch mit einem oder mehreren schmalen Längsstreifen versehen sein, welche die faserige Struktur aufweisen.

Bevorzugt ist die Klettlasche an einem dem ersten Längsende gegenüberliegenden, zweiten Längsende angeordnet. Damit liegt vorzugsweise die Klettlasche der Lasche gegenüber.

In Varianten kann auch an beiden gegenüberliegenden Längsenden eine Klettlasche vorgesehen sein. In diesem Fall kann - muss aber nicht - auf die Lasche verzichtet werden.

Vorzugsweise umfasst der Verband ein Etikett, welches insbesondere an der Klettlasche, vorzugsweise in einem Übergangsbereich zwischen Klettlasche und Bandteil angeordnet ist.

In Varianten kann auf das Etikett verzichtet werden.

Bevorzugt ist das Etikett mit Kontaktdaten für einen Notfalldienst, insbesondere einer Telefonnummer, einem QR-Code oder dergleichen versehen. Damit kann der Verwender notfalls schnell Kontakt mit einem Krankenhaus oder einem Sanitätsdienst aufnehmen. In Varianten oder zusätzlich kann das Etikett auch mit Informationen über den Hersteller, die medizinische Substanz etc. versehen sein.

Vorzugsweise umfasst der Verband mindestens einen Sensor zum Ermitteln von Daten des Patienten, insbesondere einen Pulssensor, einen Temperatursensor und/oder einen

Glukosesensor. Dem Fachmann sind weitere geeignete Sensoren bekannt. Der Verband kann weiter Mittel zum Versenden der Daten umfassen, insbesondere zum Beispiel über Bluetooth©, WLAN, Infrarot etc. Der Verband kann auch ein Speichermedium zum Speichern der gemessenen Daten umfassen.

In Varianten kann auf den Sensor verzichtet werden.

In einem Verfahren zur Herstellung eines Verbandes wird ein Bandteil in einem Webverfahren hergestellt, wobei zusammen mit einem Kettfaden mindestens ein Schmelzgarn eingearbeitet wird.

In Variante kann der Bandteil auch mit anderen Techniken hergestellt werden. Zum Beispiel kann der Bandteil durch Wirken, Stricken, Häkeln etc. hergestellt werden. Weiter können neben den Geweben auch Vliese eingesetzt werden. Der Verband kann weiter auch aus Meterware gelasert werden.

Weiter können, zum Beispiel mit der Jacquard-Technologie, Markennamen, Markierungen, Anzeigen für Festigkeit der Anbringung am Körper, etc. angebracht werden. Ein Branding oder Produkthinweise können auch mit einem Druckverfahren, Prägeverfahren oder einem ähnlichen Verfahren auf dem Bandteil aufgebracht werden. Auf solche Markierungen kann auch verzichtet werden.

Vorzugsweise wird zur Imprägnierung der Bandteil durch eine antibakterielle, flüssige Appretur geleitet und danach ein Überschuss der Appretur, insbesondere zwischen zwei Walzen, abgepresst. Damit wird ein effizientes Verfahren zur Herstellung des Verbandes geschaffen, welches insbesondere kontinuierlich betrieben werden kann.

In Varianten können auch andere Herstellungsverfahren vorgesehen sein. Insbesondere können auch vorgängig imprägnierte Fäden in einem Webvorgang etc. eingesetzt werden. Weiter können auch Silberfäden in einem Webvorgang in den Stoff eingearbeitet werden. Dem Fachmann sind weitere Techniken bekannt.

Vorzugsweise wird nach dem Abpressen der Appretur der Bandteil einem Trocknungsprozess, insbesondere bei einer Temperatur zwischen 80 °C und 190 °C unterzogen, in welchem der Schmelzgarn geschmolzen wird.

In Varianten kann der Trocknungsprozess und der Schmelzprozess auch in separaten Schritten ausgeführt werden.

Aus der nachfolgenden Detailbeschreibung und der Gesamtheit der Patentansprüche ergeben sich weitere vorteilhafte Ausführungsformen und Merkmalskombinationen der Erfindung.

### Kurze Beschreibung der Zeichnungen

Die zur Erläuterung des Ausführungsbeispiels verwendeten Zeichnungen zeigen:
- Fig. 1: eine schematische Draufsicht auf eine erste Ausführungsform eines Verbands; und
- Fig. 2: eine schematische Draufsicht auf eine zweite Ausführungsform eines Verbands.

Grundsätzlich sind in den Figuren gleiche Teile mit gleichen Bezugszeichen versehen.

### Wege zur Ausführung der Erfindung

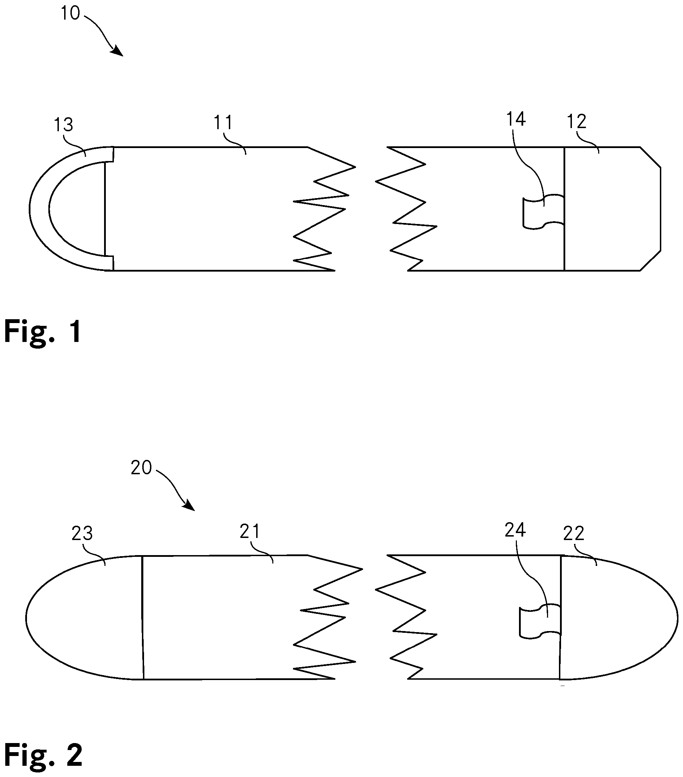

Die Figur 1 zeigt eine erste Ausführungsform eines Verbandes 10. Der Verband 10 umfasst einen Bandteil 11. Der Bandteil 11 ist streifenförmig ausgebildet und weist in der vorliegenden Ausführungsform eine Länge von 1070 mm und eine Breite von 75 mm auf. Der Bandteil weist einseitig ein VELCRO© auf und ist auf der gegenüberliegenden Seite glatt ausgeführt. In der Anwendung liegt die glatte Oberfläche des Verbandes 10 zum Körper.

Der Bandteil 11 ist vorliegend gewoben und mit einer antibakteriellen Substanz imprägniert, welche vorliegend als antibakteriellen Wirkstoff Zink umfasst. Beim Webprozess des Bandteils 11 wird in der Kette und bei jedem Kettfaden zusätzlich ein Schmelzgarn eingearbeitet. Die Webbindung im Verbund mit dem Schmelzgarn ist ausschliesslich im Kettverlauf.

Die Webbindung ist dabei so ausgebildet, dass diejenige Seite, welche auf der Haut aufliegt, glatt ist. Die gegenüberliegende Seite ist mit VELCRO© respektive Frottee-ähnlichen Schlaufen (faserig) ausgeführt, um einen gut haltenden Klettverschluss zu gewährleisten.

Für die Imprägnierung wird eine antibakterielle Appretur in einen Foulard-Trog eingelassen. Das Band wird in einem kontinuierlichen Verfahren durch die Appretur gezogen. Die überschüssige Appretur wird abgequetscht, damit eine definierte Menge auf dem Band verbleibt. In einem Trocknungsprozess bei einer Temperatur zwischen 80 °C und 190 °C, wird mit einer definierten Geschwindigkeit zwischen 3 und 25 Meter pro Minute das Band getrocknet. Dabei wird das beim Webprozess eingearbeitete Schmelzgarn verflüssigt. Anschließend verfestigt sich das Schmelzgarn während dem Abkühlen wieder. Durch das Schmelzgarn wird der Schlaufeneffekt gefestigt und stabilisiert, so dass das Vielfache Öffnen und Schliessen mit einem Klettverschluss massiv verbessert wird.

An einem ersten Längsende des Bandteils 11 ist eine Klettlasche 12 angebracht. Vorliegend ist die Klettlasche 12 als Hakenteil mit Webrand ausgebildet. Die Klettlasche weist gekappte Ecken auf. In einer weiteren Ausführungsform (nicht dargestellt) weist die Klettlasche 12 einen runden Abschluss auf (siehe dazu auch Figur 2). Die Klettlasche 12 weist eine Länge von 50 mm auf, wovon rund 6 mm mit dem Bandteil 11 überlappen. Die Breite der Klettlasche 12 ist geringfügig schmaler als diejenige des Bandteils 11. Die Breite der Klettlasche beträgt vorliegend 74 mm. In der Überlappung ist die Klettlasche 12 vorliegend mit dem Bandteil 11 verschweisst. In einer weiteren Ausführungsform (nicht dargestellt) ist die Klettlasche 12 mit dem Bandteil 11 vernäht. Die Klettlasche 12 kann mit Herstellerinformationen etc. bedruckt sein.

Zwischen der Klettlasche 12 und dem Bandteil 11 ist eine Etikette 14 befestigt. Die Etikette 14 kann mit dem Fachmann bekannten Informationen versehen sein, wie zum Beispiel einer Waschanweisung, Herstellerdaten etc. Weiter kann die Etikette 14 auch Kontaktdaten für einen Notfalldienst etc. umfassen. In der Überlappung ist die Etikette 14 vorliegend zwischen dem Bandteil 11 und der Klettlasche 12 verschweisst. In einer weiteren Ausführungsform (nicht dargestellt) ist die Etikette 14 zwischen der Klettlasche 12 und dem Bandteil 11 vernäht.

An einem zweiten, dem ersten gegenüberliegenden, Ende des Bandteils 11 ist in der vorliegenden Ausführungsform eine Schlaufe 13 respektive eine Lasche angebracht. Die Schlaufe 13 ist durch einen Stoffstreifen gebildet, welcher über deren Enden mit dem Bandteil 11 verschweisst oder, in einer weiteren Ausführungsform (nicht dargestellt) vernäht ist. Der Stoffstreifen der Schlaufe 13 weist vorliegend eine Länge von 170 mm und eine Breite von 12 mm auf. Die Schlaufenenden überlappen mit dem Bandteil 11 zu rund 6 mm und sind bündig mit den Längskanten des Bandteils 11 am Bandteil 11 befestigt. Die Schlaufe 13 kann aus demselben Material wie der Bandteil 11 bestehen, wobei die glatte Seite der Schlaufe 13 und die glatte Seite des Bandteils 11 auf derselben Seite liegen. In einer weiteren Ausführungsform (nicht dargestellt) kann die Schlaufe 13 auch aus einem anderen Material ausgebildet sein.

In einer weiteren Ausführungsform (nicht dargestellt) weist der Bandteil 11 eine Länge von 980 mm und eine Breite von 50 mm auf. Die Klettlasche 12 weist entsprechend bei einer Länge von 50 mm eine Breite von 48 mm auf.

Die Figur 2 zeigt eine zweite Ausführungsform eines Verbandes 20 mit einem Bandteil 21. Im Unterschied zur ersten Ausführungsform des Verbandes 10 umfasst der vorliegende Verband 20 der zweiten Ausführungsform keine Schlaufe, sondern an den beiden gegenüberliegenden Längsenden des Bandteils 21 jeweils eine Klettlasche 22 und 23. Die Klettlaschen 22 und 23 sind vorliegend mit dem Bandteil 21 in einem Überlappungsbereich vernäht. In einer weiteren Ausführungsform (nicht dargestellt) sind die Klettlaschen 22 und 23 mit dem Bandteil 21 in einem Überlappungsbereich verschweisst.

Der Bandteil 21 weist vorliegend eine Länge von 980 mm und eine Breite von 50 mm auf. Die beiden Klettlaschen 22, 23 weisen einen Webrand auf oder sind zugeschnitten auf eine Länge von 50 mm und eine Breite von 48 mm (geringfügig schmaler als die Breite des Bandteils 21). Die distalen Enden der Klettlaschen 22, 23 sind in Bezug auf den Verband 20 abgerundet. In einer weiteren Ausführungsform (nicht dargestellt) sind die Klettlaschen 22, 23 mit gekappten Ecken ausgeführt. Die Klettlaschen 22, 23 weisen eine Überlappung von rund 10 mm mit dem Bandteil auf. Zwischen der Klettlasche 22 und dem Bandteil 21 ist weiter eine Etikett 24 vorliegend vernäht respektive in einer weiteren Ausführungsform (nicht dargestellt) verschweisst.

Generell sind die Klettlaschen respektive die Schlaufen derart mit dem Bandteil verbunden, dass diese einer Kraft von mindestens 200 Newton, vorzugsweise mindestens 500 Newton standhalten können.

Die Abmessungen der Bandteile, der Klettlaschen und der Schlaufen können auch anderweitig gewählt sein.

Zusammenfassend ist festzustellen, dass erfindungsgemäss ein Verband geschaffen wird, welcher universell einsetzbar ist und eine besonders einfache Handhabung erlaubt, da die zur Behandlung/Therapie notwendige Substanz, insbesondere die antiseptische Substanz bereits auf dem Verband fixiert ist.

## Patentansprüche

1. Verband (10), insbesondere zur Verwendung in einem Erste-Hilfe Einsatz oder als Stützverband, umfassend einen Bandteil (11), **dadurch gekennzeichnet, dass** am Bandteil (11) zumindest bereichsweise eine medizinische, insbesondere eine antiseptische Substanz fixiert ist.

2. Verband (10) nach Anspruch 1, **dadurch gekennzeichnet, dass** der Verband (10) einen streifenförmigen Bandteil (11) und einen Verschlussteil umfasst.

3. Verband (10) nach Anspruch 1 oder 2, **dadurch gekennzeichnet, dass** der Verband (10) eine Länge zwischen 500 und 2000 mm, insbesondere zwischen 800 mm und 1400 mm, besonders bevorzugt zwischen 900 und 1200 mm aufweist.

4. Verband (10) nach einem der Ansprüche 1 bis 3, **dadurch gekennzeichnet, dass** eine Breite des Bandteils (11) zwischen 50 mm und 100 mm, insbesondere zwischen 70 mm und 80 mm liegt.

5. Verband (10) nach einem der Ansprüche 1 bis 4, **dadurch gekennzeichnet, dass** der Bandteil (11) in einer Längsrichtung dehnbar ist, insbesondere zwischen 20 % und 100 %, vorzugsweise zwischen 30 % und 70 %, insbesondere bevorzugt zwischen 45 % und 55 % bezogen auf eine Gesamtlänge des Verbandes (10).

6. Verband (10) nach einem der Ansprüche 1 bis 5, **dadurch gekennzeichnet, dass** der Bandteil (11) quersteif ist.

7. Verband (10) nach einem der Ansprüche 1 bis 6, **dadurch gekennzeichnet, dass** der Bandteil (11) ein Polyamid mit einem Anteil zwischen 80 % und 99 %, insbesondere einem Anteil zwischen 85 % und 95 % und ein Elastomer mit einem Anteil von 1 % bis 20 %, insbesondere zwischen 5 % und 15 % umfasst.

8. Verband (10) nach Anspruch 7, **dadurch gekennzeichnet, dass** das Elastomer einen Silikonfaden umfasst.

9. Verband (10) nach einem der Ansprüche 1 bis 8, **dadurch gekennzeichnet, dass** der Bandteil (11) einlagig ausgebildet ist.

10. Verband (10) nach Anspruch 2, **dadurch gekennzeichnet, dass** der Verschlussteil eine Lasche (13)umfasst, welche insbesondere als Daumenlasche und vorzugsweise längselastisch ausgebildet ist.

11. Verband (10) nach Anspruch 10 , **dadurch gekennzeichnet, dass** die Lasche (13) an einem ersten Längsende des Bandteils (11) angeordnet ist und insbesondere eine Länge zwischen 100 mm und 300 mm, insbesondere zwischen 150 mm und 190 mm und eine Breite zwischen 5 mm und 30 mm, insbesondere zwischen 10 mm und 14 mm aufweist.

12. Verband (10) nach Anspruch 2, **dadurch gekennzeichnet, dass** der Verschlussteil weiter eine Klettlasche umfasst.

13. Verband (10) nach Anspruch 12, **dadurch gekennzeichnet, dass** eine erste Hauptfläche des Bandteils (11) derart ausgebildet ist, dass sie mit der Klettlasche (12) zusammen wirken kann, wobei die erste Hauptfläche insbesondere faserig ausgebildet ist, und eine der ersten gegenüberliegende, zweite Hauptfläche derart ausgebildet ist, dass sie nicht mit der Klettlasche (12) zusammen wirken kann, wobei die zweite Hauptfläche insbesondere glatt ausgebildet ist.

14. Verband (10) nach Anspruch 12 oder 13, **dadurch gekennzeichnet, dass** die Klettlasche (12) an einem dem ersten Längsende gegenüberliegenden, zweiten Längsende angeordnet ist.

15. Verband (10) nach einem der Ansprüche 1 bis 14, **dadurch gekennzeichnet, dass** sie ein Etikett (14) umfasst, welches insbesondere an der Klettlasche (12), vorzugsweise in einem Übergangsbereich zwischen Klettlasche (12) und Bandteil (11) angeordnet ist.

16. Verband (10) nach Anspruch 15, **dadurch gekennzeichnet, dass** das Etikett (14) mit Kontaktdaten für einen Notfalldienst, insbesondere einer Telefonnummer, einem QR-Code oder dergleichen versehen ist.

17. Verband (10) nach einem der Ansprüche 1 bis 16, **dadurch gekennzeichnet, dass** er mindestens einen Sensor zum Ermitteln von Daten, insbesondere einen Pulssensor, einen Temperatursensor und/oder einen Glukosesensor umfasst.

18. Verfahren zur Herstellung eines Verbandes (10), insbesondere eines Verbandes (10) nach einem der vorherigen Ansprüchen, wobei an einem Bandteil (11) eine antiseptische Substanz fixiert wird.

19. Verfahren nach Anspruch 18, **dadurch gekennzeichnet, dass** der Bandteil (11) durch eine, die antiseptische Substanz umfassende, flüssige Appretur geleitet wird und danach insbesondere ein Überschuss der Appretur, insbesondere zwischen zwei Walzen, abgepresst wird.

20. Verfahren nach Anspruch 18, **dadurch gekennzeichnet, dass** der Bandteil (11) in einem Webverfahren hergestellt wird, wobei zusammen mit einem Kettfaden mindestens ein Schmelzgarn eingearbeitet wird.

21. Verfahren nach Anspruch 19, **dadurch gekennzeichnet, dass** nach dem Abpressen der Appretur der Bandteil (11) einem Trocknungsprozess, insbesondere bei einer Temperatur zwischen 80 °C und 190 °C unterzogen wird, in welchem das Schmelzgarn geschmolzen wird.
